# EUROPEAN PATENT SPECIFICATION

(11) **EP 2 982 355 B1**
(45) Date of publication and mention of the grant of the patent: **19.12.2018**
(21) Application number: 15179648.9
(22) Date of filing: 04.08.2015
(51) Int. Cl.: A61F 13/02, A61M 25/02

(54) **SELF-ADHESIVE DRESSING**
SELBSTKLEBENDER VERBAND
PANSEMENT AUTO-ADHÉSIF

(30) Priority: 04.08.2014 ES 201431094 U
(43) Date of publication of application: 10.02.2016
(73) Proprietor: Iberhospitex S.A., 08185 Lliça de Vall (Barcelona) (ES)
(72) Inventor: NOGUERA TORRES, Eduard, 08185 LLIÇÀ DE VALL (ES); LÓPEZ RODRÍGUEZ, Aniceto, 08185 LLIÇÀ DE VALL (ES); MAÑES AMIGÓ, Albert, 08185 LLIÇÀ DE VALL (ES)
(74) Representative: ZBM Patents - Zea, Barlocci & Markvardsen

(56) References cited:
- EP-A1- 0 671 182
- EP-A1- 1 114 651
- WO-A1-2010/049417
- CN-U- 202 699 828
- US-A- 5 415 642
- US-A1- 2003 056 794
- US-B1- 6 540 724

## Description

The present disclosure relates to a self-adhesive dressing that allows protecting a venous access device from contact with water.

### STATE OF THE ART

In medical care of patients with certain diseases it is needed, many times, the creation of a route through which the venous system can be accessed for the purpose of blood extraction, hydration of the patient, administering of medication and kidney dialysis, among others. Venous access devices are used to generate these routes, such as catheters or PICC lines. Such venous access devices typically include an insertion end that penetrates through the skin and into the patient's vein, a valve mechanism which closes the access to the vein when the device is not in use, and a connection port that allows the connection to the vein by an external device such as an IV (intravenous) drip source. These devices are inserted into a patient's vein and can remain in the body for an extended period of time, from days to months, or even years.

It is well known the risk that catheterized patients develop infectious processes due to the wounds caused by the catheterization. People carrying this type of devices should therefore fulfil very strict hygiene guidelines, in order to keep both the device and the area where it is implanted isolated, clean and dry as much as possible.

To this end, several dressings have been developed which protect and isolate the venous access device. For example, utility model ES 1043707 U describes a catheter protector made of a padded material constituting a barrier to the entry of microorganisms, the protector having at its top an adhesive strip that fixes the sheath to the skin of the patient. EP671182 discloses catheter protector for catheters of permanent or semi-permanent type arranged through the skin of a patient at an entry point, comprising a flexible bag of substantially waterproof material; an aperture in the bag surrounded by adhesive means for attaching the bag to the skin of the patient around the catheter entry point thereby providing a substantially waterproof seal around the catheter, characterized in that the bag comprises reclosable closure means for providing access to said catheter within the bag without removing the bag from the patient or, alternatively, in that the bag further comprises moisture removal means for controlling the moisture level within the bag. WO2010/049417 discloses a dressing for a catheter application, comprising: at least a surface layer for resting on the skin, the layer being permeable to blood and body fluids; at least an internal absorbent layer; wherein at least one of the surface layer and the absorbent layer comprises at least a substance selected from a group consisting of silver, aluminium and derivatives thereof, characterised in that it comprises at least a slit selected from a group consisting of: an upturned-y-shaped slit, starting from an edge of the dressing with a first cut and terminating in a bifurcation defined by two further cuts originating at an end of the first cut and identifying an angle; or fissure-shaped, starting from an edge of the dressing and terminating with a hole, which single cut is of a length comprised between 0 and 6 cm and which hole is circular with a diameter comprised between 1 .5 mm and 7 mm.

However, the commercially available dressings do not prevent the device and the area where it is implanted from getting wet when the patient takes a bath or a shower, so each time the patient is washed, medical staff has to disinfect and dry well both the skin area and the catheter, and change the dressing by another one that is dry.

Therefore, despite performed efforts up to date, a need exists for adhesive dressings that make both the catheter and the area around it effectively impermeable.

### DESCRIPTION

To solve the technical problem concerning the impermeability of self-adhesive dressings, a new dressing concept is proposed in which a sheath has been developed with a specific design and materials that attribute a hydrophobic/hydrophilic dual character thereto with two adhesive strips, one of them being impermeable and breathable. This new dressing concept isolates the venous access device from pathogens and makes it effectively impermeable.

Thus, a self-adhesive dressing for protecting a venous access device is proposed, the dressing comprising a sheath to house the venous access device in its inside, characterized in that the sheath is a breathable and impermeable sheath comprising an outer compress and an inner compress which are attached to each other forming at least one access opening to a space of the sheath that is adapted to receive the venous access device, wherein:
the outer compress has an impermeable hydrophobic outer side and a hydrophilic inner side, and
the inner compress has an impermeable hydrophobic outer side and a hydrophilic inner side,
and in that the self-adhesive dressing further comprises:
- a first adhesive strip that is impermeable and breathable and that is incorporated at the level of the opening of the sheath, and
- a second adhesive strip suitable for fixing the dressing to the skin; and
in that the inner side of the outer and inner compresses has a hydrophilic nature, absorbing any possible fluid loss in the device

Regarding the self-adhesive dressing described in ES1043707 U, the dressing proposed herein has the following advantages: (a) is impermeable to water, so that the patient may take a shower/be washed without the risk of the catheter or body area getting wet, thus minimizing the risk of infection and, on the other hand, reduces costs, since it is not necessary to replace the dressing by another one that is dry; (b) the hydrophilic component permits the absorption of the sweat as well as the possible loss of fluid by the venous access device, helping to keep the wound area dry; and (c) the inclusion of a second adhesive strip improves the adaptability to the patient because it is possible to secure the sheath of the device by both ends thereof. In addition, the self-adhesive dressing proposed herein is aimed at the protection of all types of temporary and permanent external venous access devices; and it is of easy placement for the medical staff, which prevents discomfort to the patients during its placement and removal.

In one embodiment, the sheath extends at least partially through the breathable and impermeable adhesive strip. The extension that the sheath may occupy on the adhesive strip can be any, as long as the dual function of the strip (on the one hand, adhering the sheath to the patient's body surface and, on the other hand, perfectly isolating and making the catheter opening impermeable) is not negatively affected.

Herein, the term "through" means that the sheath extends occupying the width of the first adhesive strip without exceeding its limits.

Herein, the term "inner compress" refers to the compress which is in contact with the patient's skin, while "outer compress" refers to the compress which is exposed to external agents.

Herein, the term "inner side" when referred to the inner and outer compresses, refers to the side of the compresses which, once sealed, generate the inner space of the sheath and contact the venous access device once introduced. While the term "outer side" refers to the side of the compresses that contacts the skin (in the case of the inner compress) and external agents (in the case of the outer compress).

In particular, the outer compress of the sheath is the one that extends through the breathable and impermeable adhesive strip. Again, the extension that can occupy the outer compress on the adhesive strip can be any, as long as the dual function of the strip is not negatively affected, as discussed above.

The outer and inner compresses can be made of any absorbent, biocompatible and sterilizable material. In one embodiment, the compresses are made of a combination of viscose and polyester.

In one embodiment, the inner compress, the outer compress or both compresses are padded compresses. Padding confers to the dressing, on the one hand, a high capacity of absorption, creating in turn a barrier to microorganisms and, on the other hand, an increased sensation of comfort to the patient because he no longer has the discomfort associated with frictions of the device with the skin. In another embodiment, the sheath comprises a padded inner compress and a non-padded outer sheath. In another embodiment, the sheath comprises padded inner and outer compresses.

As already indicated above, the outer side of the outer and inner compresses must be impermeable to liquids. To this end, any commercial compress having some type of polymer confering said impermeable character may be chosen. Examples of this type of polymer are: polypropylene (PP), polyethylene (PE), polyurethane (PU), polyester, Polyethylene terephthalate (PET), Ethylene-vinyl acetate (EVA), polyethers and polytetrafluoroethylene (PTFE). In one embodiment, the outer side of the outer and inner compresses comprises polypropylene.

The inner side of the outer and inner compresses has a hydrophilic nature, so this material can absorb any possible fluid loss in the device. To achieve this hydrophilic character, the skilled person may use commercial fibres such as viscose, cellulose fibre, cotton, polyamides (PA), polylactic acid and polyacrylic acid. In one embodiment, the hydrophilic inner side of the compresses comprises viscose.

The two compresses can be attached to each other, leaving at least one opening suitable for housing the venous access device, by using methods well known to the skilled person in the art such as ultrasonic sealing, by thermal lamination, by adhesive, by needling. In one embodiment, the two compresses are sealed by ultrasound.

One of the improvements incorporated in the dressing that is proposed herein, is the incorporation of a breathable and impermeable adhesive strip. In one embodiment, this strip is transparent. Thus, it can be visually detected from the outside, without having to take off the dressing, whether the area of application of the venous access device has turned red, which is an indication of an infection onset. The breathable and impermeable adhesive strip may be a film of polyester or polyurethane. The adhesive applied to this strip is a pressure sensitive and moisture resistant adhesive. Examples of adhesive with this profile are acrylic adhesives (also called polyacrylates), polyurethanes adhesives and silicones. In one embodiment, the breathable and impermeable adhesive strip is made of polyurethane. The first adhesive strip may have any size provided that it effectively covers the opening of the sheath and has a sufficient area to ensure that the dressing remains attached. In one embodiment, this first adhesive strip comprises in the upper part a strip of polyethylene having appropriate dimensions to allow the user to properly handle the dressing.

Another improvement incorporated in the dressing proposed herein is the incorporation of a second adhesive strip. In one embodiment, this second adhesive strip is arranged on an opposite side of the sheath relative to the first adhesive strip. This second adhesive strip can be made of any material suitable for contacting the skin such as a film, a fabric, or a non-woven fabric, among others, whose chemical nature may be, for example, polyurethane, polyester, polypropylene or polyethylene, among others. This strip is made adherent with an acrylic adhesive. In one embodiment the second adhesive strip is made of a non-woven fabric of polyester made adherent with an acrylic adhesive. In another embodiment, the second strip may be a polyethylene film with an acrylic adhesive. The acrylic adhesive is characterized by a smooth and stable adherence to the skin, is hypoallergenic, permeable to water vapour and resistant to moisture.

In another embodiment, the second adhesive strip is supported on a strip of non-woven fabric. Thus, the adhesive strip has a greater adaptability to the area of application. Furthermore, such type of fabric is characterized by having high porosity, which confers a higher breathability to the strip.

In another embodiment, the second adhesive strip is arranged on an opposite side of the sheath relative to the first adhesive strip, is made of polyacrylate and is supported on non-woven fabric.

In another embodiment the adhesive strips are provided with protective sheets. These protective sheets may be based on wax paper, siliconized paper or the like, so that, for the implementation of the protector, it is enough the coupling of the sheath in such a way that the venous access device remains housed therein is enough, to a limit situation in which the adhesive strip, after removal of the protective sheets, is fixed to the patient's body acting as fastening means of the protector as a whole, and as sealing means of the opening of the sheath. For removal of the protector, peeling off the adhesive strip is enough, so that the sheath can be easily removed by a simple axial displacement relative to the device.

In another embodiment, the venous access device is a catheter. Catheters are tubes that are inserted into the large venous vessels of the chest or into the right heart chambers, with diagnostic or therapeutic purposes. Illustrative and non-limiting examples of catheters are the reservoir catheter, Hickman catheter and PICC catheter (peripherally inserted central catheter).

In one embodiment, the self-adhesive dressing proposed herein is for protecting a catheter, the dressing comprising: (a) a breathable and impermeable sheath comprising an outer and an inner compress, said compresses being sealed so as to form an access opening to an interior space of the sheath which is adapted to receive the device, wherein the inner and outer compresses have a hydrophobic impermeable outer side comprising polypropylene and a hydrophilic inner side comprising viscose and cellulose fibres; (b) a first impermeable and breathable adhesive strip made of polyurethane; and (c) a second adhesive strip of polyester non-woven fabric made adherent with a polyacrylate adhesive, this second strip being on an opposite side of the sheath relative to the first adhesive strip.

In one embodiment the dressing is sterilized by any of the known sterilization methods for this type of products. These methods comprise the sterilization through ethylene oxide and beta or gamma radiation.

In one embodiment the self-adhesive dressing is incorporated in a leak-proof envelope.

In another embodiment the self-adhesive dressing is sealed within a leak-proof envelope that has been sterilized by a treatment with ethylene oxide.

In another embodiment the self-adhesive dressing is incorporated in a leak-proof envelope that has been sterilized by gamma or beta radiation.

### BRIEF DESCRIPTION OF THE DRAWINGS

In the following, a particular embodiment of an example of this self-adhesive dressing for the protection of venous access devices is described. This description is provided by way of non-limiting example and with reference to the accompanying drawings. In the drawings:
Figure 1 is a top perspective view of an embodiment of the self-adhesive dressing for the protection of venous access devices;
Figure 2 is a plan view of the dressing of Figure 1 which illustrates how the catheter would be arranged inside the sheath.

### DETAILED DESCRIPTION OF AN EMBODIMENT

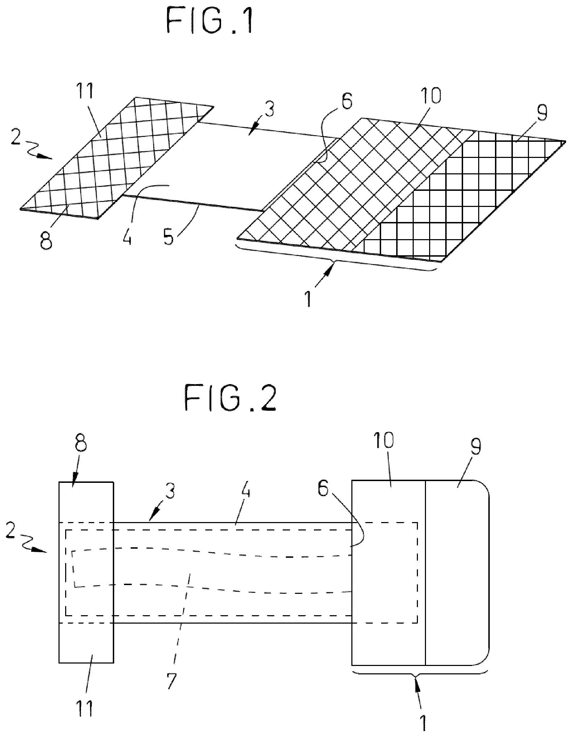

In the particular non-limiting example shown in Figures 1 and 2, the self-adhesive dressing has been generally designated by the reference 2. The dressing 2 is constituted by an impermeable and breathable sheath 3 formed by two compresses, an inner compress 4 and an external compress 5. The inner and outer compresses are made of a combination of viscose and polyester and have an outer impermeable hydrophobic side comprising polypropylene and a hydrophilic inner side comprising viscose and cellulose fibres. The two compresses are sealed forming an access opening 6 and an inner space of the sheath which is adapted to receive the venous access device 7 as shown in Figure 2. The dressing includes, at the level of the opening of the sheath, a breathable and impermeable polyurethane adhesive strip 1. As shown in Figure 1, the outer compress 5 extends through the polyurethane strip 1 to achieve a higher sealing effect. On the opposite side of the sheath 3 with respect to the polyurethane adhesive strip 1, the dressing has an adhesive strip 8 made of a material suitable for skin contact and made adherent with an acrylic adhesive that helps to better fix the device to the patient's skin. The adhesive strip 8 is supported on a strip of non-woven fabric. Adhesive strips 1 and 8 are provided with protective films 9-11.

For use, the venous access device is inserted into the breathable and impermeable sheath 3 through the access opening 6. Then, the bottom protector 10 of the breathable and impermeable adhesive strip 1 is removed and is fixed to the patient's body in the proper position; and then the adhesive protectors 9 and 11 are removed, leaving the dressing completely fixed to the patient's body. Figure 3 shows how the catheter 7 is disposed inside the sheath.

The self-adhesive dressing is commercialized packaged in a leak-proof manner, so that it keeps its sterility characteristics until its use.

Within the scope of the present invention, the skilled person will also be able to introduce modifications and technically equivalent elements. For example, other adhesive strips can be incorporated to the dressing that is proposed herein in order to improve its capacity of fixation. These strips may be supported along the protective sheath of the venous access device.

The present invention covers all the possible combinations of the particular embodiments that have been described. The scope of the present invention should not be limited to particular embodiments, but it should be determined only by a proper reading of the appended claims.

## Claims

1. Self-adhesive dressing for protecting a venous access device, the dressing comprising a sheath to house the venous access device in its inside, **characterized in that** the sheath is a breathable and impermeable sheath comprising an outer compress and an inner compress which are attached to each other forming at least one access opening to a space of the sheath that is adapted to receive the venous access device, wherein:
the outer compress has an impermeable hydrophobic outer side and a hydrophilic inner side, and
the inner compress has an impermeable hydrophobic outer side and a hydrophilic inner side,
**in that** the self-adhesive dressing further comprises:
- a first adhesive strip that is impermeable and breathable and that is incorporated at the level of the opening of the sheath, and
- a second adhesive strip suitable for fixing the dressing to the skin; and
**in that** the inner side of the outer and inner compresses has a hydrophilic nature, absorbing any possible fluid loss in the device.

2. Self-adhesive dressing according to claim 1 **characterized in that** the sheath extends at least partially through the breathable and impermeable adhesive strip.

3. Self-adhesive dressing according to claim 2, **characterized in that** the outer compress of the sheath is the compress which extends through the breathable and impermeable adhesive strip.

4. Self-adhesive dressing according to claim 1 **characterized in that** the outer and inner compresses comprise a mixture of viscose and polyester.

5. Self-adhesive dressing according to claim 1 **characterized in that** the inner compress, the outer compress or both are padded compresses.

6. Self-adhesive dressing according to claim 1 **characterized in that** the impermeable hydrophobic outer side of the outer and inner compresses comprises polypropylene.

7. Self-adhesive dressing according to claim 1 **characterized in that** the hydrophilic inner side of the outer and inner compresses comprises viscose and cellulose fibres.

8. Self-adhesive dressing according to claim 1 **characterized in that** the outer and inner compresses are sealed.

9. Self-adhesive dressing according to claim 1 **characterized in that** the first breathable and impermeable adhesive strip is transparent.

10. Self-adhesive dressing according to claim 9 **characterized in that** the first breathable and impermeable adhesive strip is made of polyurethane.

11. Self-adhesive dressing according to claim 1 **characterized in that** the second adhesive strip is arranged on an opposite side of the sheath relative to the first adhesive strip.

12. Self-adhesive dressing according to claim 1 or 11 **characterized in that** the second adhesive strip is made of polyester non-woven fabric or polyethylene film with an acrylic adhesive.

13. Self-adhesive dressing according to any of claims 1, 9 to 12 **characterized in that** the adhesive strips are provided with protective sheets.

14. Self-adhesive dressing according to any of the preceding claims, **characterized in that** the venous access device is a catheter.

15. Self-adhesive dressing according to claim 1, for protecting a catheter, the dressing comprising: (a) a breathable and impermeable sheath comprising an outer and an inner compress, said compresses being sealed so as to form an access opening to an interior space of the sheath which is adapted to receive the device, wherein the inner and outer compresses have a hydrophobic impermeable outer side comprising polypropylene and a hydrophilic inner side comprising viscose and cellulose fibres; (b) a first impermeable and breathable adhesive strip made of polyurethane; and (c) a second adhesive strip made of polyester nonwoven fabric made adherent with a polyacrylate adhesive, this second strip being on an opposite side of the sheath relative to the first adhesive strip.

16. Self-adhesive dressing according to any of the preceding claims that is incorporated in a leak-proof envelope.

17. Self-adhesive dressing according to claim 16 **characterized in that** it is sterilized.

18. Self-adhesive dressing according to claim 17 **characterized in that** it is sterilized by ethylene oxide.

19. Self-adhesive dressing according to claim 17 **characterized in that** it is sterilized by a treatment with gamma or beta radiation.

## Patentansprüche

1. Selbstklebender Verband zum Schutz von einer Venenzugangsvorrichtung, wobei der Verband eine Hülse umfasst, um die Venenzugangsvorrichtung darin aufzunehmen, **dadurch gekennzeichnet, dass** die Hülse eine atmungsaktive und undurchlässige Hülse ist, die eine äußere Kompresse und eine innere Kompresse umfasst, die an einander befestigt sind und mindestens eine in einen Raum der Hülse führende Zugangsöffnung bilden, welcher Raum angepasst ist, um die Venenzugangsvorrichtung aufzunehmen, wobei:
die äußere Kompresse eine undurchlässige hydrophobe äußere Seite und eine hydrophile innere Seite hat, und
die innere Kompresse eine undurchlässige hydrophobe äußere Seite und eine
hydrophile innere Seite hat,
dadurch, dass der selbstklebender Verband weiterhin folgendes umfasst:
- einen ersten Klebestreifen, der undurchlässig und atmungsaktiv ist und der auf der Ebene der Öffnung der Hülse integriert ist, und
- einen zweiten Klebestreifen, der geeignet ist, um der Verband an der Haut fest zu halten; und
dadurch, dass die innere Seite der äußeren und der inneren Kompressen hydrophiler Natur ist und jeden möglichen Flüssigkeitsverlust in der Vorrichtung absorbiert.

2. Selbstklebender Verband nach Anspruch 1, **dadurch gekennzeichnet, dass** sich die Hülse mindestens teilweise durch den atmungsaktiven und undurchlässigen Klebestreifen erstreckt.

3. Selbstklebender Verband nach Anspruch 2, **dadurch gekennzeichnet, dass** die äußere Kompresse der Hülse die Kompresse ist, die sich durch den atmungsaktiven und undurchlässigen Klebestreifen erstreckt.

4. Selbstklebender Verband nach Anspruch 1, **dadurch gekennzeichnet, dass** die äußere und innere Kompresse jeweils eine Mischung aus Viskose und Polyester umfassen.

5. Selbstklebender Verband nach Anspruch 1, **dadurch gekennzeichnet, dass** die innere Kompresse, die äußere Kompresse oder beide wattierte Kompressen sind.

6. Selbstklebender Verband nach Anspruch 1, **dadurch gekennzeichnet, dass** die undurchlässige hydrophobe äußere Seite der äußeren und inneren Kompressen Polypropylen umfasst.

7. Selbstklebender Verband nach Anspruch 1, **dadurch gekennzeichnet, dass** die hydrophile innere Seite der äußeren und inneren Kompressen Viskose- und Cellulosefasern umfasst.

8. Selbstklebender Verband nach Anspruch 1, **dadurch gekennzeichnet, dass** die äußere und innere Kompressen versiegelt sind.

9. Selbstklebender Verband nach Anspruch 1, **dadurch gekennzeichnet, dass** der erste atmungsaktive und undurchlässige Klebestreifen durchsichtig ist.

10. Selbstklebender Verband nach Anspruch 9, **dadurch gekennzeichnet, dass** der erste atmungsaktive und undurchlässige Klebestreifen aus Polyurethan besteht.

11. Selbstklebender Verband nach Anspruch 1, **dadurch gekennzeichnet, dass** der zweite Klebestreifen auf einer gegenüberliegenden Seite der Hülse bezüglich des ersten Klebestreifens angeordnet ist.

12. Selbstklebender Verband nach Anspruch 1 oder 11, **dadurch gekennzeichnet, dass** der zweite Klebestreifen aus Polyester-Vliesstoff oder aus Polyethylenfilm mit einem Acrylklebstoff besteht.

13. Selbstklebender Verband nach einem der Ansprüche 1, 9 bis 12, **dadurch gekennzeichnet, dass** die Klebstreifen mit Schutzabdeckfolien versehen sind.

14. Selbstklebender Verband nach einem der vorhergehenden Ansprüche, **dadurch gekennzeichnet, dass** die Venenzugangsvorrichtung ein Katheter ist.

15. Selbstklebender Verband nach Anspruch 1 zum Schutz von einem Katheter, wobei der Verband folgendes umfasst: (a) eine atmungsaktive und undurchlässige Hülse umfassend eine äußere und eine innere Kompresse, wobei die Kompressen versiegelt sind, um eine in einen inneren Raum der Hülse führende Zugangsöffnung zu bilden, der angepasst ist, um die Vorrichtung aufzunehmen, wobei die innere und äußere Kompresse jeweils eine hydrophobe undurchlässige äußere Seite haben, die Polypropylen umfasst und eine hydrophile innere Seite umfassend Viskose- und Cellulosefasern haben; (b) einen ersten undurchlässigen und atmungsaktiven Klebestreifen, der aus Polyurethan besteht; und (c) einen zweiten Klebestreifen, der aus Polyester-Vliesstoff besteht, der mittels eines Polyacrylatklebstoffs haftend gemacht worden ist, wobei dieser zweiter Streifen auf einer gegenüberliegenden Seite der Hülse bezüglich des ersten Klebestreifens angeordnet ist.

16. Selbstklebender Verband nach einem der vorhergehenden Ansprüche, der in einem lecksicheren Umschlag integriert ist.

17. Selbstklebender Verband nach Anspruch 16, **dadurch gekennzeichnet, dass** er sterilisiert worden ist.

18. Selbstklebender Verband nach Anspruch 17, **dadurch gekennzeichnet, dass** er mittels Ethylenoxid sterilisiert worden ist.

19. Selbstklebender Verband nach Anspruch 17, **dadurch gekennzeichnet, dass** er mittels einer Behandlung mit Gamma- oder Betastrahlung sterilisiert worden ist.

## Revendications

1. Pansement autoadhésif pour protéger un dispositif d'accès veineux, le pansement comprenant une gaine pour héberger le dispositif d'accès veineux dans son intérieur, **caractérisé en ce que** la gaine est une gaine respirante et imperméable comprenant une compresse extérieure et une compresse intérieure qui sont attachées l'une à l'autre formant au moins une ouverture d'accès à un espace de la gaine qui est adapté pour recevoir le dispositif d'accès veineux, dans lequel :
la compresse extérieure a un côté extérieur hydrophobe imperméable et un côté intérieur hydrophile, et
la compresse intérieure a un côté extérieur hydrophobe imperméable et un côté intérieur hydrophile,
**en ce que** le pansement autoadhésif comprend en outre :
- une première bande adhésive qui est imperméable et respirante et qui est incorporée au niveau de l'ouverture de la gaine, et
- une deuxième bande adhésive appropriée pour fixer le pansement à la peau ; et
**en ce que** le côté intérieur des compresses extérieure et intérieure est de nature hydrophile, absorbant tout perte de fluide possible dans le dispositif.

2. Pansement autoadhésif selon la revendication 1 **caractérisé en ce que** la gaine s'étend au moins en partie par la bande adhésive respirante et imperméable.

3. Pansement autoadhésif selon la revendication 2, **caractérisé en ce que** la compresse extérieure de la gaine est la compresse qui s'étend par la bande adhésive respirante et imperméable.

4. Pansement autoadhésif selon la revendication 1 **caractérisé en ce que** les compresses extérieure et intérieure comprennent un mélange de viscose et polyester.

5. Pansement autoadhésif selon la revendication 1 **caractérisé en ce que** la compresse intérieure, la compresse extérieure ou toutes deux sont des compresses matelassées.

6. Pansement autoadhésif selon la revendication 1 **caractérisé en ce que** le côté extérieur hydrophobe imperméable des compresses extérieure et intérieure comprend du polypropylène.

7. Pansement autoadhésif selon la revendication 1 **caractérisé en ce que** le côté intérieur hydrophile des compresses extérieure et intérieure comprend des fibres de viscose et de cellulose.

8. Pansement autoadhésif selon la revendication 1 **caractérisé en ce que** les compresses extérieure et intérieure sont scellées.

9. Pansement autoadhésif selon la revendication 1 **caractérisé en ce que** la première bande adhésive respirante et imperméable est transparente.

10. Pansement autoadhésif selon la revendication 9 **caractérisé en ce que** la première bande adhésive respirante et imperméable est faite en polyuréthane.

11. Pansement autoadhésif selon la revendication 1 **caractérisé en ce que** la deuxième bande adhésive est disposée sur un côté opposé de la gaine par rapport à la première bande adhésive.

12. Pansement autoadhésif selon la revendication 1 ou 11 **caractérisé en ce que** la deuxième bande adhésive est faite en tissu non tissé de polyester ou en film de polyéthylène avec un adhésif acrylique.

13. Pansement autoadhésif selon l'une quelconque des revendications 1, 9 à 12 **caractérisé en ce que** les bandes adhésives sont munies de feuilles de protection.

14. Pansement autoadhésif selon l'une quelconque des revendications précédentes, **caractérisé en ce que** le dispositif d'accès veineux est un cathéter.

15. Pansement autoadhésif selon la revendication 1, pour protéger un cathéter, le pansement comprenant : (a) une gaine respirante et imperméable comprenant une compresse extérieure et une compresse intérieure, étant lesdites compresses scellées pour former une ouverture d'accès à un espace intérieur de la gaine qui est adapté pour recevoir le dispositif, dans lequel les compresses intérieure et extérieure ont un côté extérieur imperméable hydrophobe comprenant du polypropylène et un côté intérieure hydrophile comprenant des fibres de viscose et de cellulose ; (b) une première bande adhésive imperméable et respirante faite en polyuréthane ; et (c) une deuxième bande adhésive faite en tissu non tissé de polyester fait adhérent avec un adhésif de polyacrylate, cette deuxième bande étant sur un côté opposé de la gaine par rapport à la première bande adhésive.

16. Pansement autoadhésif selon l'une quelconque des revendications précédentes qui est incorporé dans une enveloppe étanche.

17. Pansement autoadhésif selon la revendication 16, **caractérisé en ce qu'**il est stérilisé.

18. Pansement autoadhésif selon la revendication 17, **caractérisé en ce qu'**il est stérilisé avec de l'oxyde d'éthylène.

19. Pansement autoadhésif selon la revendication 17, **caractérisé en ce qu'**il est stérilisé par traitement moyennant du rayonnement gamma ou béta.
